# EUROPÄISCHE PATENTSCHRIFT

(11) **EP 1 448 220 B1**
(45) Veröffentlichungstag und Bekanntmachung des Hinweises auf die Patenterteilung: **09.07.2008**
(21) Anmeldenummer: 02792816.7
(22) Anmeldetag: 27.11.2002
(51) Int. Cl.: A61K 38/01, A61K 35/20, A61K 31/00, A61P 29/00

(54) **PHARMAZEUTISCHES MITTEL ENTHALTEND EINEN ANTIPHLOGISTISCHEN WIRKSTOFF UND LACTALBUMINHYDROLYSAT UND DESSEN VERWENDUNG**
PHARMACEUTICAL COMPOSITION COMPRISING AN ANTIPHLOGISTIC AGENT AND LACTALBUMINHYDROLYSATE AND THE USE THEREOF
AGENT PHARMACEUTIQUE CONTENANT UN AGENT ANTIPHLOGISTIQUE ET UN HYDROLYSAT DE LACTALBUMIN ET SON UTILISATION

(30) Priorität: 27.11.2001 DE 10158039
(43) Veröffentlichungstag der Anmeldung: 25.08.2004
(73) Patentinhaber: Exalation LTD, Elstree Herts WD6 3EW (GB)
(72) Erfinder: GÖRNE, Martin, 22337 Hamburg (DE)
(74) Vertreter: Reitstötter - Kinzebach
(86) Internationale Anmeldenummer: PCT/EP2002/013385
(87) Internationale Veröffentlichungsnummer: WO 2003/045416

(56) Entgegenhaltungen:
- EP-A- 0 282 020
- WO-A-86/04217
- WO-A-99/03451
- GB-A- 1 460 108

## Beschreibung

Die vorliegende Erfindung betrifft pharmazeutische Mittel enthaltend antiphlogistische Wirkstoffe und Lactalbuminhydrolysat sowie deren Verwendung zur Behandlung von Erkrankungen des rheumatischen Formenkreises.

Lactalbuminhydrolysate werden seit einiger Zeit als Diätnahrungsmittel verwendet. Beispielsweise wird ein Peptidgemisch aus Lactalbumin, Fleisch und Soja bei Maldigestion, Malabsorption, bei konsumierenden Prozessen und zum Aufbau verwendet. Lactalbuminhydrolysate zeichnen sich dadurch aus, dass sie sehr leicht aufgenommen werden können und dem Stoffwechsel in kürzester Zeit zur Verfügung stehen. Insbesondere die niedermolekulare Fraktion ist für die Resorption und weitere Verwertung im Stoffwechsel besonders gut geeignet.

Darüber hinaus wurde auch bereits darüber berichtet, dass ausgehend von Molkenprotein, Lactalbumin, α-Lactalbumin, Lactoferrin, β-Lactoglobulin, Lysozym oder Serumalbumin Abbauprodukte gewonnen werden können, die pharmakologische Wirkungen besitzen sollen. So erwähnen die DE-A 39 22 453 und die PCT/EP86/00016 (WO 86/04217) beispielsweise eine analgetische, antiphlogistische oder antimutagene Wirksamkeit. Es wird daher empfohlen, die Abbauprodukte zur Behandlung von schmerzhaften Entzündungen aller Art, Neurodermitis, Arthritis, Rheuma, aber auch von Glaukomen zu verwenden. Auch die gemäß DE 38 29 552 A1 erhältlichen Milchbestandteile sollen charakteristische pharmakologische Eigenschaften besitzen und zur Behandlung von Neurodermitis, Allergien, Glaukomen und zur Immunstimulierung brauchbar sein. Allerdings ist die pharmakologische Wirksamkeit dieser Lactalbuminhydrolysate heftig umstritten. Sie konnte auch in der für die klinische Anwendung geforderten Weise nicht belegt werden.

Werden chronisch entzündlich verlaufende Erkrankungen wie beispielsweise bestimmte rheumatische Erkrankungen mit steroidalen und nichtsteroidalen Antiphlogistika behandelt, so sind möglichst geringe Wirkstoffdosen wünschenswert, um die mit der in der Regel erforderlichen Langzeitbehandlung verbundenen Nebenwirkungen auf ein erträgliches Maß zu reduzieren. Beispielsweise kann im Rahmen einer Corticoid-Therapie ein iatrogen induziertes Cushing-Syndrom auftreten, oder es können sich Geschwüre des Duodenums und der Ventriculums ausbilden. Was nicht-steroidale Antiphlogistika angeht, ist wegen der Heterogenität der zugrunde liegenden Stoffklassen eine Vielzahl unerwünschter Wirkungen bekannt. Beispielsweise können Phenylbutazone eine Agranulocytose oder Ödeme, Acetylsalicylsäure, Arylessigsäure-Derivate und Arylpropionsäure-Derivate Magenblutungen, Pyrazolone eine Cytopenie, Anthranilsäure-Derivate eine Glumerulonephritis, Azapropazon Nierenfunktionsstörungen, Piroxicam und Tenoxicam Nierenfunktions- und Darmfunktionsstörungen, Paracetamol Leberschädigungen, Goldverbindungen und Methotrexat Nierenschädigungen, und Chloroquin Einlagerungen und Veränderungen an der Hornhaut des Auges verursachen.

Der Erfindung liegt daher die Aufgabe zugrunde, möglichst nebenwirkungsarme antiphlogistische Mittel zur Verfügung zu stellen.

Es wurde nun gefunden, daß die Wirkung bekannter antiphlogistischer Wirkstoffe erheblich verstärkt werden kann, wenn man sie in Kombination mit Lactalbuminhydrolysaten verabreicht.

Gegenstand der vorliegenden Erfindung sind daher pharmazeutische Mittel enthaltend i) wenigstens einen antiphlogistischen Wirkstoff und ii) Lactalbuminhydrolysat oder eine Lactalbuminhydrolysat-Peptiden enthaltende Fraktion davon.

Die erfindungsgemäßen Mittel bieten wesentliche therapeutische Vorteile. Insbesondere können die Antiphlogistika wesentlich geringer dosiert werden als es herkömmlicherweise erforderlich ist, um eine bestimmte antiphlogistische Wirkung zu erzielen. In der Regel kann die Dosierung des Antiphlogistikums unter Beibehaltung seiner antiphlogistischen Hauptwirkung halbiert oder gar noch weiter reduziert werden. Dadurch treten weniger, auf das Antiphlogistikum zurückzuführende Nebenwirkungen auf. Dies ist insbesondere dann von Bedeutung, wenn eine antiphlogistische Behandlung über einen längeren Zeitraum angezeigt ist, wie beispielsweise bei der Behandlung chronischer rheumatischer Erkrankungen. Ermöglicht wird dies dadurch, dass die Lactalbuminhydrolysate die antiphlogistische Wirkung der Antiphlogistika verstärken und damit die Menge, die zur Erzielung einer bestimmten antiphlogistischen Wirkung benötigt wird, vergleichsweise niedriger ist.

Die Antiphlogistika und die Lactalbuminhydrolysate können prinzipiell gemeinsam in einer Formulierung oder getrennt in wenigstens zwei verschiedenen Formulierungen verabreicht werden. Die letztere Möglichkeit beinhaltet sowohl die gleichzeitige als auch die zeitlich beabstandete, d.h. zu unterschiedlichen Zeitpunkten erfolgende, Verabreichung. Bevorzugt ist die gleichzeitige Verabreichung insbesondere in Form einer gemeinsamen Formulierung.

Lactalbumine sind allgemein als Bestandteil der Milch und insbesondere der Molkenproteine bekannt. Es handelt sich hierbei um Proteine, die in an sich bekannter Weise meist im Gemisch mit weiteren Milchbestandteilen, also insbesondere Molkenproteinen, wie Lactalbumin, Lactoferrin, β-Lactoglobulin, Lysozym oder Serumalbumin, gewonnen werden können.

Als Ausgangsmaterial wird frische Rohmilch eines Haustieres, vorzugsweise Kuhmilch verwendet, die auch einer in den Molkereien heute üblichen Erhitzungsbehandlung unterworfen worden sein kann, vorzugsweise aber unbehandelt ist. Sie kann in üblicher Weise, z.B. durch Zentrifugieren, entrahmt worden sein.

Die Abtrennung der Kaseine kann in an sich bekannter Weise erfolgen, beispielsweise mit Hilfe der sogenannten Lab- oder Säurefällung, bei der die Kaseine ausfallen, während die Molkenproteine in Lösung bleiben. Alternativ können die Kaseine mit Hilfe einer Membranfiltration an einer mikroporösen Membran mit einer Porengröße im Bereich von 0,1 bis 0,6 µm, vorzugsweise 0,2 µm abgetrennt werden. Das Permeat (bzw. Filtrat) enthält sämtliche Salze, Milchzucker, Aminosäuren, Oligopeptide und niedermolekulare Peptide, während im Retentat praktisch alle Kaseinbestandteile der Milch und - sofern nicht vorher entrahmt - auch deren Fettbestandteile enthalten sind.

Aus der von Kasein und Fett weitgehend befreiten Milchfraktion können dann Lactalbumine in an sich bekannter Weise, insbesondere durch Ultrafiltration, gewonnen werden. Beispielsweise können die Lactalbumine als Retentat an einer Membran mit einer Abscheidegrenze von 6 bis 10.000 Da erhalten werden. Brauchbar ist beispielsweise das von der Firma Milei vertriebene Produkt Lactalbumin 75.

Einem besonderen Aspekt zufolge werden erfindungsgemäß bevorzugt Lactalbuminhydrolysate oder Fraktionen davon verwendet, deren gewichtsmittleres Molekulargewicht in einem Bereich von etwa 50 bis 1200, vorzugsweise von etwa 60 bis 800 und insbesondere von etwa 80 bis 500 liegt. Besonders bevorzugt sind Lactalbuminhydrolysate, deren Molekulargewichtsverteilung durch ein oberes Molekulargewicht von etwa 760 und insbesondere von 500 begrenzt ist.

Das erfindungsgemäß zu verwendende Lactalbuminhydrolysat ist dadurch erhältlich, dass man Lactalbumin oder ein Lactalbumin enthaltendes Gemisch (Lactalbumin-Präparation) enzymatisch hydrolisiert. Dazu behandelt man eine wässrige Lactalbumin-Suspension mit wenigstens einer Protease und gewünschtenfalls einer Lipase.

Die Hydrolyse erfolgt in der Regel vollständig, d.h. bis zur Beendigung der enzymatischen Reaktion. Je nach Enzym oder Enzymgemisch kann ein Hydrolysegrad von etwa 20% bis etwa 90%, vorzugsweise von etwa 40% bis etwa 80% und insbesondere etwa 75% erzielt werden.

Der Hydrolysegrad kann in an sich bekannter Weise bestimmt und berechnet werden. So ist es möglich, den Fortschritt der Hydrolyse während der Reaktion zu verfolgen. Als geeignet hat sich insbesondere das Formol-Titrationsverfahren erwiesen, wonach die Anzahl freier Aminogruppen durch Titration mit Natriumhydroxid abgeschätzt werden kann.

Die zwecks Hydrolyse wählbaren Bedingungen, wie die eingesetzten Enzyme und deren Aktivität, die Temperatur, der pH-Wert, die Menge an Lactalbumin und die jeweiligen Konzentrationen des Reaktionsgemisches hängen voneinander ab und können auf fachmännische Art und Weise optimiert werden.

Als Protease(n) verwendet man vorzugsweise Papain, Pankreatin, Chymotrypsin und/oder Trypsin. Gegebenenfalls können auch aus Pilzen und/oder Bakterien gewonnene Proteasen eingesetzt werden, insbesondere zusätzlich zu den vorstehend genannten Proteasen. Die Pilzprotease wird insbesondere ausgewählt unter den Proteasen aus Tritirachium-Arten, insbesondere Tritirachium alba, Proteasen aus Aspergillus-Arten, insbesondere Aspergillus saitoi, Aspergillus sojae, Aspergillus oryzae und/oder aus Rhizopus-Arten, insbesondere Newlase, und die Bakterienprotease wird insbesondere ausgewählt unter Proteasen aus Streptomyces-Arten, insbesondere Streptomyces caespitosus, Streptomyces griseus (Pronase E.), aus Bacillus subtilis-Arten, insbesondere Subtilopeptidase A (Carlsberg Subtilisin), und aus Bacillus polymyxa. Als weitere Proteasen können aus Ananas gewinnbare Proteasen wie das Bromelain verwendet werden.

Sollte die Lactalbumin-Präparation noch merkliche Beimengungen an Stärke und stärkeähnlichen Produkten enthalten, ist es vorteilhaft, eine geeignete Amylase, vorzugsweise eine α-Amylase z.B. aus einer Subtilis-Art mitzuverwenden. Das Aktivitätsoptimum einer derartigen Amylase liegt in der Regel bei einem pH-Wert von 5,7 bis 7,2. Die Reaktionstemperatur kann bis zu 75°C betragen.

Die Mitverwendung einer Lipase ist in der Regel auch nur dann erforderlich, wenn die Lactalbumin-Präparation merkliche Beimengungen an Fetten enthält.

Vorteilhafterweise werden die Enzyme in einer Menge von etwa 0,01 bis 2 Gew.-%, bezogen auf die Suspension, verwendet.

Als Protease verwendet man vorzugsweise Papain oder ein Gemisch von etwa gleichen Gewichtsteilen Pankreatin und Papain.

Ein weiteres vorteilhaftes Proteasengemisch ist ein Gemisch aus etwa gleichen Anteilen Papain, Pankreatin und einer Bakterien- oder Pilzprotease, z.B. der Bakterienprotease Pronase E aus Streptomyces griseus oder dem Pilzprotease-Produkt "Newlase" aus einer Aspergillus-Art. Ebenfalls von Vorteil ist ein Proteasengemisch aus etwa gleichen Anteilen Papain, Pankreatin und Bromelain.

Insbesondere ist es von Vorteil, bezogen auf etwa 50 kg Lactalbumin als Trockensubstanz, etwa 100 bis 1000 g, vorzugsweise etwa 300 bis 700 g und insbesondere etwa 500 g Papain; etwa 100 bis 1000 g, vorzugsweise etwa 300 bis 700 g und insbesondere etwa 500 g Pankreatin; sowie gegebenenfalls etwa 100 bis 1000 g, vorzugsweise etwa 300 bis 700 g insbesondere etwa 500 g einer Bakterien- oder Pilzprotease, insbesondere Newlase, bzw. einer aus Ananas gewinnbaren Protease, insbesondere Bromelain, zu verwenden.

Zweckmäßigerweise geht man von einer etwa 1 bis 10 gew.%-igen und vorteilhafterweise einer 2 bis 5 gew.%-igen Suspension von Lactalbumin in Wasser aus.

Üblich sind Reaktionszeiten im Bereich von Stunden, insbesondere etwa 1 bis 8 Stunden und vorteilhafterweise etwa 2 bis 4 Stunden; erhöhte Reaktionstemperaturen, etwa 30 bis 70°C und vorteilhafterweise im Bereich des Temperaturoptimums der eingesetzten Enzyme; pH-Werte im leicht sauren bis leicht alkalischen Bereich, insbesondere etwa bei 6,6 bis 7,8 und vorzugsweise im pH-Optimum der eingesetzten Enzyme. Bei Verwendung von Enzym-Gemischen und insbesondere solchen mit unterschiedlichen Temperatur- bzw. pH-Optima, kann man die jeweiligen Enzyme zu unterschiedlichen Zeiten auf das Lactalbumin einwirken lassen, indem man einen Teil des Enzymgemisches zu einem späteren Zeitpunkt dem Reaktionsgemisch zusetzt und die Bedingungen entsprechend anpasst. Insbesondere kann man die wärmebeständigeren Enzyme zugeben, nachdem man zunächst bei moderater Temperatur unter Einsatz der weniger wärmebeständigen Enzyme das Lactalbumin zumindest teilweise hydrolisiert hat, und anschießend die Hydrolyse unter Verwendung der wärmebeständigeren Enzyme bei höherer Temperatur fortsetzten. So kann man im vorliegenden Fall zunächst bei Temperaturen im Bereich von 35 bis 38 °C und vorzugsweise bei etwa 37°C Papain und/ oder Pankreatin auf das Lactalbumin einwirken lassen und anschließend die Hydrolyse unter Einwirkung einer geeigneten Bakterien- und/oder Pilzprotease bzw. einer aus Ananas gewinnbaren Protease bei Temperaturen im Bereich von etwa 50 bis 75 °C und vorzugsweise bei etwa 60 °C fortsetzen. Zweckmäßigerweise geht man dazu so vor, dass die Protease dem Reaktionsgemisch zugesetzt und dann die Temperatur allmählich erhöht wird. Insbesondere kann es erwünscht sein, die Temperatur während der noch verbleibenden Reaktionszeit graduell auf die Endtemperatur zu erhöhen.

So wird gemäß einer besonderen Ausführungsform der vorliegenden Erfindung ein Lactalbuminhydrolysat verwendet, das dadurch erhältlich ist, dass man ein Papain und Pankreatin enthaltendes Enzymgemisch bei etwa 35 bis 38 °C 1 bis 3 Stunden auf Lactalbumin in wässriger Suspension einwirken lässt; eine Bakterien- oder Pilzprotease bzw. eine aus Ananas gewinnbare Protease zugibt und die Temperatur innerhalb von 1 bis 4 Stunden auf etwa 60 °C erhöht und das Hydrolysat in an sich bekannter Weise gewinnt.

Zur Gewinnung des Hydrolysats geht man zweckmäßigerweise so vor, dass man zunächst die Enzyme inaktiviert. Dazu kann man das Reaktionsgemisch kurz erhitzen. Üblich sind Temperaturen im Bereich von 80 bis 85 °C, wobei eine Inaktivierung schon nach wenigen Minuten erzielt wird. Alternativ können die Enzyme auch durch Sterilisierung bei Ultrahochtemperaturen inaktiviert werden. Wenige Sekunden bei etwa 130 °C reichen im allgemeinen aus.

Als Folge fallen die Enzyme in der Regel aus und der entstandene Niederschlag wird vorzugsweise nach dem Abkühlen des Reaktionsgemisches entfernt. Dazu kann man das Reaktionsgemisch direkt filtrieren oder zunächst einengen, beispielsweise im Vakuum oder durch Sprühtrocknung, um den Rückstand dann in einem geeigneten Lösungsmittel aufzunehmen und die resultierende Suspension zu filtrieren. Als Lösungsmittel eignen sich in diesem Zusammenhang niedere Alkohole wie Methanol, Isopropanol und vorzugsweise Ethanol bzw. deren Gemische mit Wasser und auch organische Lösungsmittel, insbesondere Chloroform.

Aus 5 g Lactalbumin erhält man auf diese Weise beispielsweise etwa 200 mg Lactalbuminhydrolysat in ethanolischer Lösung, wobei dieses Hydrolysat häufig als Gemisch aus den eigentlichen Lactalbuminpeptiden als Hauptbestandteil und weiteren Nebenbestandteilen anfällt. Art und Menge der Nebenbestandteile, insbesondere Fette, hängen vor allem von dem für die Hydrolyse verwendeten Ausgangsmaterial ab.

Gemäß einer besonderen Ausführungsform der vorliegenden Erfindung verwendet man bestimmte Fraktionen aus Lactalbuminhydrolysaten, die insbesondere aus den zuvor beschriebenen durch Extraktion erhältlich sind.

Insbesondere bevorzugt ist es, vor der Extraktion der Lactalbuminhydrolysate diese zunächst zu entfetten. Hierzu eignen sich in bekannter Weise Lösungsmittel wie beispielsweise Petroläther, die zweckmäßigerweise in einer kontinuierlichen, erschöpfenden Extraktion zur Entfernung der Fettbestandteile eingesetzt werden können.

Als Extraktionsmittel eignen sich vorwiegend polare Lösungsmittel, wie niedere Alkohole, insbesondere Methanol, Ethanol, Isopropanol und deren Gemische mit Wasser.

Bevorzugte Fraktionen sind dadurch erhältlich, dass man Lactalbuminhydrolysat mit absolutem Ethanol extrahiert, filtriert und das Filtrat gewinnt. Die ethanolische Lösung enthält eine Lactalbuminhydrolysat-Fraktion A (Ethanol-Extrakt), die durch Einengen als Feststoff gewonnen werden kann. Diese Extraktion des vorzugsweise entfetteten Lactalbuminhydrolysats mit Ethanol erfolgt vorzugsweise durch kontinuierliche, erschöpfende Extraktion. Dies beinhaltet auch die Abtrennung nicht extrahierbarer, d.h. in Ethanol unlöslicher Bestandteile unter Gewinnung einer Extraktlösung, die anschließend zur Trockne eingeengt werden kann, um die auf diese Art extrahierbare Fraktion des Lactalbuminhydrolsyats als Feststoff zu gewinnen.

Besonders bevorzugte Fraktionen sind dadurch erhältlich, daß man Lactalbuminhydrolysat oder Fraktionen davon mit Isopropanol extrahiert, filtriert und das Filtrat gewinnt. Die isopropanolische Lösung enthält eine Lactalbuminhydrolysat-Fraktion B (Isopropanol-Extrakt), die durch Einengen als Feststoff gewonnen werden kann. Ein bevorzugter Isopropanol-Extrakt ist dadurch erhältlich, dass man den mittels Ethanol-Extraktion erhaltenen Rückstand A einsetzt und entsprechend mit Isopropanol extrahiert (Lactalbuminhydrolysat-Fraktion B1).

Dazu kann Lactalbuminhydrolysat und insbesondere die mittels der zuvor beschriebenen Ethanolextraktion gewonnene Fraktion A in Isopropanol aufgenommen werden, so dass ein gut rührbares Gemisch entsteht. Beispielsweise kann das Lactalbuminhydrolysat bzw. eine Fraktion davon mit etwa dem 10-fachen Volumen Isopropanol verrührt werden. In der Regel erhält man eine Suspension, die zur Abtrennung von Feststoffen filtriert wird. Es hat sich als zweckmäßig erwiesen, das Gemisch zunächst einige Stunden bis wenige Tage vorzugsweise in der Kälte aufzubewahren und dann die resultierende Feststoffphase abzufiltrieren. Etwa 2 Tage bei einer Temperatur von 2 °C bis 4 °C führen zu guten Ergebnissen. Anschließend wird das Filtrat zur Trockne eingeengt, wobei obigen Ausführungen zur Einengung des Ethanolextraktes entsprechend verfahren werden kann. Darüber hinaus hat es sich als zweckmäßig erwiesen, zur Entfernung von Isopropanolresten den Rückstand wiederholt in Ethanol aufzunehmen und durch Einengen von Lösungsmitteln zu befreien. Beispielsweise werden durch dreimaliges Aufnehmen mit ausreichend Ethanol und anschließendem Einengen gute Ergebnisse erzielt.

Besonders bevorzugte Fraktionen sind dadurch erhältlich, daß man den mittels Ethanol-Extraktion erhaltenen Rückstand A in absolutem Ethanol aufnimmt und mit Wasser einen Ethanolgehalt von etwa 20 bis 60 Vol.-%, vorzugsweise von etwa 30 bis 50 Vol.-% und insbesondere von etwa 40 Vol.-%, einstellt. In der Regel erhält man eine Suspension, deren Feststoffphase durch Filtration abgetrennt werden kann. Dazu ist es zweckmäßig, das gegebenenfalls zunächst noch als Lösung vorliegende Gemisch mehrere Stunden bis wenige Tage, vorzugsweise in der Kälte aufzubewahren und dann abzufiltrieren. Auch hier führen 2 Tage bei 2 °C bis 4 °C zu guten Ergebnissen. Die wäßrig-ethanolische Lösung enthält eine Lactalbuminhydrolysat-Fraktion C, die durch Einengen als Feststoff gewonnen werden kann. Bevorzugte Lactalbuminhydrolysat-Fraktionen sind erhältlich, indem man den mittels Isopropanol-Extraktion erhaltenen Rückstand B und insbesondere B1 einsetzt und entsprechend verfährt, wobei man eine Lactalbuminhydrolysat-Fraktion C1 bzw. insbesondere eine Lactalbuminhydrolysat-Fraktion C2 gewinnt.

In der Regel erfolgt das Einengen zur Trockne in an sich bekannter Weise, z.B. im Vakuum oder durch Sprühtrocknung. Das Einengen kann zweckmäßigerweise durch Verdampfen des Ethanols bei leicht erhöhter Temperatur, beispielsweise bei etwa 40 °C, erfolgen. Gewünschtenfalls wird der gewonnene Rückstand weiter getrocknet, beispielsweise im Vakuum. Andere, ähnlich schonende Verfahren sind ebenfalls geeignet. Für die Entfernung des Isopropanols bzw. wäßrig-alkoholischer Gemische gilt ähnliches.

Die Lactalbuminhydrolysat-Fraktionen sind gekennzeichnet durch einen erhöhten Gehalt an Lactalbuminhydrolysat-Peptiden, einer Fraktion des anfänglich eingesetzten Lactalbuminhydrolysats. Einem besonderen Aspekt zufolge beinhaltet diese Peptidfraktion 2 Petide (Peptid A und Peptid B), deren jeweiliger Anteil an der Peptidfraktion größer ist als der Anteil eines beliebigen weiteren Peptids in dieser Fraktion. Insbesondere handelt es sich bei den beiden Peptiden A und B um Tri- und/oder Tetrapetide. Einem weiteren Aspekt zufolge sind die Peptide A und B durch ein chromatographisches Verhalten gekennzeichnet, wonach sie mittels Umkehrphasen-Chromatographie, z.B. bei Verwendung einer RP 18-Säule (5 µ-Material) durch Elution mit einem Acetonitril/Wasser/Trifluoressigsäure-Gradienten, bei ausreichend unterschiedlichen Verweilzeiten, aufgereinigt werden können.

Dementsprechend können die Peptide A und B aus Lactalbuminhydrolysaten oder Fraktionen davon angereichert und gewonnen werden.

Besondere Lactalbuminhydrolysat-Peptide erfindungsgemäßer Lactalbuminhydrolysate sind einem weiteren Aspekt zufolge gekennzeichnet durch ein Molekülion-Peak (m + H⁺) bei massenspektrometischer TOF-Analyse von m/z = 247, 269, 341 und/oder 399. Von besonderer Bedeutung sind die Peptide mit den Molekülion-Peaks 341 und 399. Besondere Lactalbuminhydrolysat-Peptide sind daher vor allem auszuwählen unter Tripeptiden mit zwei Prolin-Resten sowie Glutamin oder Lysin als weiteren Aminosäurerest und Tetrapeptiden mit einem Leucin- oder einem Isoleucinrest, und einem Prolin-, einem Alanin- und einem Valinrest.

Einem Aspekt zufolge sind überwiegende Anteile erfindungsgemäß zu verwendender Lactalbuminhydrolysate oder Fraktionen davon sowohl in Chloroform als auch in Wasser leicht löslich. Diese Anteile betragen vorzugsweise wenigstens 90 Gew.-%, vorzugsweise wenigstens 95 Gew.-% und insbesondere wenigstens 98 Gew.-% des jeweiligen Lactalbuminhydrolysats bzw. der Fraktion davon.

Zu Antiphlogistika im weitesten Sinne gehören Wirkstoffe, die Entzündungen aller Art entgegenwirken. Diese werden oftmals eingeteilt in nicht-steroidale Antiphlogistika, die im wesentlichen in die Prostaglandinsynthese eingreifen, und steroidale Antiphlogistika, die gemeinhin auch als Corticosteroide bezeichnet werden. Zu den Antiphlogistika gehören insbesondere die Antirheumatika.

Besondere erfindungsgemäß verwendbare antiphlogistische Wirkstoffe sind ausgewählt unter:
a) Substanzen pflanzlichen Ursprungs, häufig in Form von Gemischen, z.B. Flavonen, Heparinen, Terpenen und Glykosiden, wie Auszügen, Extrakten, insbesondere Trockenextrakten und Tinkturen von Kamillenblüten, Bromelaine, Weidenrinde, Arnika, Herba Symphyti, Radix Symphyti, Peregrini recentis, Rosskastaniensamen;
b) Corticoiden, insbesondere Mineralcorticoiden, wie Fludrocortison und Derivaten davon, gegebenenfalls in Salzform, z.B. Fludrocortisonacetat, und Glukocorticoiden, wie Dexametason, Hydrocortison, Methylprednisolon, Prednisolon, Prednison, Triamcinolon, Deflazacort, Betamethason, Predmyliden, Budesonid, Chloprednol, Fluorcortolon, Cortison, Rimexolon, Flumetason, Flunisolid, Fluocortinbotyl, Fluocinolon, Fluocortolon, Fluorometholon, Fluticason, Beclomeson sowie Derivaten davon, gegebenenfalls in Salzform, z.B. Dexamethason-21-(3-sulfobenzoat)-Natriumsalz, Dexamethason-21-dihydrogenphosphat-Dinatriumsalz, Hydrocortison-21-hydrogensuccinat-Natriumsalz, Methylprednisolon-21-hydrogensuccinat-Natriumsalz, Prednisolon-21-dihydrogenphosphat-Dinatriumsalz, Prednisolon-Acetat, Prednisolon-21-hydrogensuccinat-Natriumsalz, Triamcinolonacetonid-dihydrogenphosphat-Dikaliumsalz, Fluticason-17-propionat, Cortison-Acetat und Beclomeson-Dipropionat;
c) Salicylsäure und Derivaten davon, gegebenenfalls in Salzform, z.B. Acetylsalicylsäure, DL-Lysinmono(acetylsalicylat);
d) Phenylbutazonen, wie Mofebutazon und Kebuzon sowie Derivaten davon, gegebenenfalls in Salzform, z.B. Phenylbutazon-Natrium, Mofebutazon-Natrium und Kebuzon-Natrium;
e) Pyrazolonen, wie Phenazon und Metamizol sowie Derivaten davon, gegebenenfalls in Salzform, z.B. Metamizol-Natrium und Propyphenazon;
f) Paracetamol;
g) Anthranilsäure-, Arylessigsäure-, und Arylpropionsäure-Derivaten, wie Acemetazin, Ibuprofen, Naproxen, Diclofenac, Indometacin, Lonazolac, Aceclophenac, Fluorbiprofen, Ketoprofen, Naproxen, Mefenaminsäure, Acemetacin, Proglumetazin, Etofenamat, Tiaprofensäure und Dexketoprofen sowie Derivaten davon, gegebenenfalls in Salzform, z.B. Ibuprofen-Lysinsalz, Naproxen-Natrium, Diclofenac-Natrium, Lonazolac-Calcium, Ipuprofen-Natriumsalz-Dihydrat, Proglumetazin-Dimaleat, Dexketoprofen-Tromethamol und Diclofenac-Cholestyramin;
h) Azapropazon, Lornoxicam, Meloxicam, Piroxicam und Tenoxicam sowie Derivaten davon, gegebenenfalls in Salzform;
i) Goldverbindungen, z.B. Auranophin und Natriumaurothiomalat, Schwefelpräparate, Sulfasalazin, Resochin, Chloroquin, Dapson und Methotrexat und Derivate davon, gegebenenfalls in Salzform, z.B. Methotrexat-Dinatrium, Penicillamin, Hydroxychloroquinsulfat;
j) Mesalacin und Cinnaricin, und Derivaten davon, gegebenenfalls in Salzform;
k) Dimethylsulfoxid;
   insbesondere
l) selektive Inhibitoren der Cyclooxygenase 1, wie Diclofenac, Ibuprofen, Ketoprofen, Tiaprofensäure, Indometacin, Acemetacin, Acetylsalizylsäure, Proglumetacin, Lonazolac, Naproxen, Piroxicam, Oxyphenbutazon, Phenylbutazon, Tenoxicam und Derivate davon, gegebenenfalls in Salzform;
m) selektiven Inhibitoren der Cyclooxygenase 2, z.B. Meloxicam, Nabumeton und Derivaten davon, gegebenenfalls in Salzform.

### Erfindungsgemäß bevorzugt sind folgende Antiphlogistika:

Prednison, Diclofenac, Ibuprofen, Indometacin und Derivate davon, gegebenenfalls in Salzform.

Erfindungsgemäß wird dem zu behandelnden Individuum, vorzugsweise einem Säuger, insbesondere einem Menschen und auch einem Nutz- oder Haustier, eine wirksame Menge Lactalbuminhydrolysat oder einer Fraktion davon und eine wirksame Menge wenigstens eines Antiphlogistikums, in der Regel der pharmazeutischen oder tierarzneilichen Praxis entsprechend formuliert, verabreicht.

In der Regel wird täglich, einmalig oder mehrmalig eine geeignete Dosis gegebenenfalls zusammen oder im Wechsel mit anderen Wirkstoffen oder wirkstoffhaltigen Präparaten verabreicht, so dass einem zu behandelnden Individuum eine Tagesdosis von etwa 2 mg bis 100 g, vorzugsweise von etwa 10 mg bis 80 g, vorteilhafterweise von etwa 20 mg bis 60 g, und insbesondere von etwa 50 mg bis 60 g Lactalbuminhydrolysat bei oraler Gabe, von etwa 0,5 mg bis 80 g, vorzugsweise von etwa 8 mg bis 70 g, vorteilhafterweise von etwa 20 mg bis 70 g, und insbesondere von etwa 30 mg bis 40 g Lactalbuminhydrolysat bei parenteraler Gabe und von etwa 0,5 mg bis 100 g, vorzugsweise von etwa 1 mg bis 80 g, vorteilhafterweise von etwa 5 mg bis 60 g, und insbesondere von etwa 10 mg bis 60 g Lactalbuminhydrolysat bei topischer Anwnendung verabreicht wird. Die zu verabreichenden Mengen an Lactalbuminfraktionen liegen in der Regel unter den vorstehend genannten Dosierungen. So kann die Dosierung für die Lactalbuminhydrolysatbestandteile etwa um den Faktor 5 bis 20 und die Dosierung für die Lactalbuminhydrolysatpeptide etwa um den Faktor 10 bis 30 niedriger gewählt werden.

Dabei kann die Dosierung des Antiphlogistikums erfindungsgemäß niedriger gewählt werden als es herkömmlicherweise zum Erzielen der antiphlogistischen Hauptwirkung empfohlen wird. Demnach kann insbesondere die zur Behandlung von Entzündungen, vor allem im Rahmen von Erkrankungen des rheumatischen Formenkreises, empfohlene Tagesdosis zumindest um den Faktor 2 und vorteilhafterweise zumindest um den Faktor 5 gesenkt werden. Werden beispielsweise einem Asthmatiker im Schub täglich 40 mg Prednison verabreicht, um sein Asthma kompensiert zu halten, so kann erfindungsgemäß die gleiche therapeutische Wirkung mit einer Kombination aus 10 mg Lactalbuminhydrolysac-Fraktion C1 oder C2 und nur 7,5 mg Prednison erzielt werden. Entsprechend könnte eine tägliche Dosis von 20 mg Prednison durch kombinierte Verabreichung von 10 mg Lactalbuminhydrolysat-Fraktion C1 oder C2 auf nur 3,5 bis 4 mg Prednison gesenkt werden.

Das Ausmaß, in dem die Dosis eines Wirkstoffs mit anerkannter antiphlogistischer Wirkung herabgesetzt werden kann, wenn dieser erfindungsgemäß in Kombination mit Lactalbuminhydrolysat oder einer Fraktion davon verwendet wird, kann insbesondere mit kontrollierten, randomisierten Doppelblindstudien belegt werden. Dazu können beispielsweise klinische Messgrößen herangezogen werden, welche den Verlauf der Therapie des untersuchten Krankheitsbildes nach wissenschaftlichen Erkenntnissen dokumentieren. Ferner können auch Tiermodelle verwendet werden.

Beispielsweise wird die Therapie bei erfindungsgemäßer Verwendung einer Kombination aus Diclofenac und einer Lactalbuminhydrolysat-Fraktion einerseits und einer 10-fach höheren Diclofenac-Dosis ohne Verwendung einer erfindungsgemäßen Lactalbuminhydrolysat-Fraktion für die Behandlung der seropositiven chronischen Polyarthritis (PCP) mit der laborchemischen Verlaufskontrolle der CRP-Werte (C-reaktives Protein), der Blutkörperchensenkungsgeschwindigkeit (BSG) und der Dokumentation der Schmerzempfindung anhand von Scores bewertet. Der Grad der Dosisherabsetzung ergibt sich unter Bezugnahme auf Referenzwerte für die Therapie der PCP.

Wirkstoffmengen und -anteile beziehen sich auf den aktiven Wirkstoff, so dass für Salze und Derivate eine entsprechende Umrechnung zu erfolgen hat.

Die erfindungsgemäßen Mittel eignen sich vor allem zur Behandlung von Entzündungen aller Art. Damit betrifft die vorliegende Erfindung auch die Verwendung von Lactalbuminhydrolysaten oder Fraktionen davon in Kombination mit wenigstens einem Antiphlogistikum zur Behandlung von Entzündungen aller Art.

Einem besonderen Aspekt zufolge können Erkrankungen des rheumatischen Formenkreises behandelt werden. Hierzu gehören insbesondere Erkrankungen des Bewegungsapparats, wie Verschleißerscheinungen, degenerative Vorgänge und entzündliche Zustände der Gelenke, Wirbelsäule, Muskeln und Sehnen, z.B. akute Affektionen des Bewegungsapparates wie Bursitis, Tendinitis, Synovitis, Tendovaginitis, Periarthritis der Schulter, Lumbalgien, Verstauchungen und Zerrungen, schmerzhafte Schwellungen oder Entzündungen nach Verletzungen oder Operationen, rheumatische Beschwerden, wie Arthrosen, Reizzustände bei Arthrosen und Spondylarthrosen, akute und chronische Arthriden, Polyarthritis, rheumatoide Arthritis, Gicht, Morbus Bechterew, Weichteilrheumatismus, oberflächliche Thrombophlebitis.

Ein besonderer Aspekt einer Behandlung im erfindungsgemäßen Sinne betrifft die Behandlung chronischer Störungen.

Die Erfindung betrifft auch die Herstellung von Mitteln zur Behandlung eines Individuums, vorzugsweise eines Säugers, insbesondere eines Menschen und auch eines Nutz- oder Haustieres.

Erfindungsgemäße Mittel basieren in der Regel auf einer Wirkstoffkombination und gegebenenfalls einer Formulierungsgrundlage.

Die Formulierungsgrundlage erfindungsgemäßer Formulierungen enthält physiologisch akzeptable Hilfsstoffe. Physiologisch akzeptabel sind die im Bereich der Pharmazie, der Lebensmitteltechnologie und angrenzenden Gebieten bekanntermaßen verwendbaren Hilfstoffe, insbesondere die in einschlägigen Arzneibüchern (z.B. DAB, Ph. Eur., BP, NF) gelisteten, und auch andere Hilfstoffe, deren Eigenschaften einer physiologischen Anwendung nicht entgegenstehen.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Cantor-Verlag, 1996, dargestellt ist.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen. Auch implantierte Abgabevorrichtungen können zur Verabreichung erfindungsgemäßer Wirkstoffe verwendet werden. Ferner können auch Liposomen oder Mikrosphären zur Anwendung kommen.

Die Formulierungen können beispielsweise auf oralem, rektalem, topischem, insbesondere transdermalem, parenteralem, insbesondere subkutanem, intravenösem, intramuskulärem, oder intranasalem Weg verabreicht werden. Bevorzugt ist die orale, topische und erforderlichenfalls auch intravenöse Verabreichung.

Bei der Herstellung der Formulierungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvörgangen, durchgeführt werden, z.B. eine Granulierung, Komprimierung und ähnliches.

Insbesondere können die Wirkstoffkomponenten gemeinsam formuliert werden. Sie können aber auch zunächst getrennt verarbeitet und anschließend in einer kompartimentierten, z.B. mehrschichtigen Arzneiform zusammengeführt werden. Dadurch kann möglichen Wirkstoffinkompatibilitäten und unterschiedlichen Wirkstoffeigenschaften, wie Bioverfügbarkeit, Stabilität, Löslichkeit und ähnlichem, Rechnung getragen werden.

Neben Kombinationspräparaten betrifft die Erfindung auch entsprechende Monopräparate in Form von Handelspackungen, denen die erfindungsgemäße kombinierte Anwendung zu entnehmen ist.

Die vorliegende Erfindung wird anhand der nachfolgenden Beispiele näher erläutert, ohne darauf beschränkt zu sein.

### Herstellung von Lactalbuminhydrolysat und Fraktionen davon

### Beispiel 1

### Herstellung eines Lactalbuminhydrolysats

5 g Lactalbumin werden in 130 ml Wasser suspendiert, man gibt 50 mg Papain und 50 mg Pankreatin hinzu, erwärmt auf 35-37 °C, rührt bei dieser Temperatur etwa 2 Stunden, gibt 50 mg einer handelsüblichen Pilzprotease, z.B. Newlase, eine Protease aus Rhizopus mit einer Aktivität von ungefähr 0,5 E/mg Masse, hinzu und steigert die Temperatur langsam (innerhalb von 2-3 Stunden) auf 60 °C. Dann erhöht man die Temperatur kurz auf 80 °C, lässt abkühlen, engt die trübe Lösung im Vakuum ein, nimmt den Rückstand in Ethanol auf, filtriert und engt im Vakuum ein. Man erhält etwa 0,2 g Lactalbuminhydrolysat.

### Beispiel 2

### Entfettung des Lactalbuminhydrolysats

100 g des nach Beispiel 1 erhaltenen Lactalbuminhydrolysats werden in einer kontinuierlichen, erschöpfenden Reaktion mit 2 1 Petroläther entfettet bei einer Temperatur von etwa 22 °C.

### Beispiel 3

### Gewinnung einer Lactalbuminhydrolysat-Fraktion A

5 g des nach Beispiel 2 erhaltenen, entfetteten Lactalbuminhydrolysats werden mit 100 ml absolutem Ethanol bei einer Temperatur von 22 °C in einer kontinuierlichen, erschöpfenden Reaktion extrahiert. Der Ethanolextrakt wird bei 40 °C eingedampft und im Vakuum getrocknet.

### Beispiel 4

### Gewinnung einer Lactalbuminhydrolysat-Fraktion B

500 g der nach Beispiel 3 erhaltenen Lactalbuminhydrolysat-Fraktion A werden mit dem 10-fachen Volumen Isopropanol verrührt und 2 Tage bei einer Temperatur von 2 °C bis 4 °C aufbewahrt. Man filtriert oder zentrifugiert und engt die klare Lösung bei 50 °C im Vakuum zur Trockne ein.

Zur Entfernung von Isopropanolresten wird der Rückstand in 2 1 Ethanol aufgenommen und das Ethanol bei 40 °C im Vakuum verdampft. Dieser Vorgang wird 2-3-mal wiederholt. Man erhält ca. 400 g einer Lactalbuminhydrolysat-Fraktion B1 als gelbliches Pulver.

### Beispiel 5

### Gewinnung einer Lactalbuminhydrolysat-Fraktion C

Die von Isopropanolresten nach Beispiel 4 befreite Lactalbuminhydrolysat-Fraktion B1 wird in 2 1 absolutem Ethanol gelöst und durch Zugabe von Wasser auf einen Ethanolgehalt von 40 Vol.-% eingestellt. Anschließend wird die Lösung 2 Tage bei 2-4 °C aufbewahrt. Man filtriert oder zentrifugiert und engt die erhaltene klare Lösung im Vakuum bei 40 °C zur Trockne ein. Man erhält ca. 300 g einer Lactalbuminhydrolysat-Fraktion C2 als gelbliches Pulver.

Die massenspektrometische Analyse der Lactalbuminhydrolysat-Fraktion C2 ergibt folgende Peaks (m/z +/- 0,5; TOF MS ES+):
72,09; 86,10; 98,99; 120,09; 127,08; 149,03; 185,18; 188,08; 199,19; 229,17; 244,18; 247,12; 269,17; 286,99; 288,98; 341,23; 349,01; 360,21; 399,27; 431,25; 453,23; 469,23; 496,35; 525,36; 527,37; 557,35; 565,28; 569,30; 587,26; 587,28; 609,38; 649,35; 654,41; 682,42; 683,36; 723,37; 724,36; 763,40; 764,43; 772,42; 773,44; 797,42; 837,47; 838,49; 860,45; 871,52; 872,51; 889,54; 926,55; 927,51; 948,52; 949,46; 951,53.

Die HPLC-analytische Untersuchung (Umkehrphase RP-18; 5 µ-Material; Elution mit einem Acetonitril/Wasser/Trifluoressigsäure-Gradienten; Detektion bei 205 nm) der Lactalbuminhydrolysat-Fraktion C2 zeigt neben freien Aminosäuren ein Peptidgemisch, das im Oligopeptid-Bereich (Dipeptide bis Decapeptide) zwei Hauptfraktionen umfasst, die, bezogen auf die bei etwa 7,5 min eluierenden hydrophoben Aminosäuren, insbesondere Tryptophan, bei etwa 16 min bzw. etwa 19,5 min eluieren. Diese Hauptfraktionen stehen ihrem chromatographischen Verhalten nach für Tri- oder Tetrapeptide, so dass anzunehmen ist, dass ein wesentlicher Bestandteil der erfindungsgemäßen Lactalbuminhydrolysate aus zwei Peptiden gebildet wird, wobei es sich um Tri- und/oder Tetrapeptide handeln dürfte.

Diese Peptide lassen sich mit einer entsprechenden präparativen HPLC isolieren.

Pharmazeutische Mittel

### Beispiel 6

### a) Weichgelatine-Kapsel mit Diclofenac

### Füllung:

| | |
|---|---|
| Diclofenac | 15 mg |
| Lactalbuminhydrolysat-Fraktion nach Bsp. 5 | 15 mg |
| Sojaöl (raff.) | 440 mg |
| Sojalecithin (E322) | 50 mg |
| Hochdisperses Siliciumdioxid Kapselhülle: | 5 mg |
| Gelatine | 303 mg |
| Glycerol 85 % | 87 mg |
| Sorbitol 70 % | 77 mg |
| gereinigtes Wasser | 52 mg |
| Eisenoxidpigment Braun 75 ( E 172) | 3 mg |

Zur Behandlung von PCP sind 1 bis 2 Kapseln 1-mal täglich zu empfehlen.

### b) Tablette mit Ibuprofen

| | |
|---|---|
| Ibuprofen | 40 mg |
| Lactalbuminhydrolysat-Fraktion nach Bsp. 5 | 20 mg |
| Milchzucker | 127,5 mg |
| Magnesiumstearat | 5 mg |
| Talcum | 23,75 mg |
| Mikrokristalline Cellulose | 81 mg |

Zur Behandlung von rheumatischer Erkrankungen sind 1 bis 2 Tabletten täglich zu empfehlen.

### c) Weiterhin können gemäß b) hergestellte Tabletten in bekannter Weise mit einem Magen- oder Dünndarm-löslichen Filmüberzug versehen werden.

### Beispiel 7

### Ibuprofen-Studie:

5 Patienten mit Colitis ulcerosa (Morbus Crohn), die regelmäßig zwischen 1800 mg und 2400 mg Ibuprofen nahmen, wurden zweimal täglich mit 50 mg Ibuprofen und 10 mg Lactalbuminhydrolysat-Fraktion gemäß Beispiel 5 erfindungsgemäß behandelt. Der Behandlungserfolg wurde anhand des CRP-Werts, der BSG und der Schmerzempfindung in Scores beurteilt. Alle 5 Fälle wurden erfolgreich eingestellt.

Wurde den Patienten lediglich die Lactalbuminhydrolysat-Fraktion, nicht aber das Ibuprofen verabreicht, war keine Besserung feststellbar, sondern der Befund verschlechterte sich wie ohne Therapie. Behandelte man dieselben Patienten im Anschluss daran mit der erfindungsgemäßen Kombination aus 500 mg Ibuprofen und 10 mg Lactalbuminhydrolysat-Fraktion jeweils zweimal täglich, konnte innerhalb von maximal 4 Tagen eine Besserung der Beschwerden festgestellt werden.

### Beispiel 8

### Diclofenac-Studie:

4 Patienten mit seropositiver Polyarthritis (PCP), die regelmäßig zwischen 100 mg und 150 mg Diclofenac nahmen, wurden mit einer Kombination aus 10 mg Diclofenac und 10 mg Lactalbuminhydrolysat-Fraktion gemäß Beispiel 5 erfindungsgemäß behandelt. Der Behandlungserfolg wurde anhand des CRP-Werts, der BSG und der Schmerzempfindung in Scores beurteilt. Die im Rahmen der erfindungsgemäßen Kombinationstherapie ermittelten Messgrößen entsprachen in einem Fall denjenigen Werten, die der Patient bei Behandlung mit 100 mg Diclofenac zeigte, in drei Fällen denjenigen Werten, die die Patienten bei Behandlung mit 150 mg Diclofenac zeigten.

Wurde den Patienten lediglich die Lactalbuminhydrolysat-Fraktion, nicht aber das Diclofenac verabreicht, trat in allen Fällen das Vollbild der Beschwerden nach durchschnittlich 6,5 Tagen wieder auf. Bei erneuter Therapie mit 10 mg Diclofenac und 10 mg Lactalbuminhydrolysat-Fraktion, stellte man innerhalb von durchschnittlich 2,5 Tagen in allen Fällen eine deutliche Besserung des Krankheits- und Beschwerdebildes fest.

### Beispiel 9

### Prednison-Studie:

9 Patienten mit allergischem Asthma bronchiale, denen regelmäßig eine Tagesdosis zwischen 20 mg und 40 mg Prednison verabreicht wurde, wurden mit einer erfindungsgemäßen Kombination aus 5 mg Prednison und 10 mg Lactalbuminhydrolysat-Fraktion gemäß Beispiel 5 täglich erfindungsgemäß behandelt.

In 8 Fällen waren die Patienten innerhalb kurzer Zeit beschwerdefrei. In einem Fall musste die Prednison-Dosis auf 20 mg täglich heraufgesetzt werden. Dieser Patient war zuvor mit einer Tagesdosis von 40 mg behandelt worden.

Wurde den Patienten lediglich die Lactalbuminhydrolysat-Fraktion, nicht aber das Prednison verabreicht, so musste diese Therapie nach durchschnittlich 2,5 Tagen abgebrochen werden, weil das Vollbild der Asthmabeschwerden, wie sonst nur bei Patienten ohne Therapie, wieder auftrat. Die erneute Therapie mit 5 mg Prednison und 10 mg Lactalbuminhydrolysat-Fraktion führte zu einer Besserung der Beschwerden innerhalb von 2 Tagen.

## Patentansprüche

1. Pharmazeutisches Mittel, enthaltend i) wenigstens einen antiphlogistischen Wirkstoff und ii) Lactalbuminhydrolysat oder eine Lactalbuminhydrolysat-Peptideenthaltende Fraktion davon.

2. Pharmazeutisches Mittel nach Anspruch 1, **dadurch gekennzeichnet, daß** das Lactalbuminhydrolysat oder die Fraktion davon ein gewichtsmittleres Molekulargewicht von etwa 50 bis 1200, vorzugsweise von etwa 60 bis 800 und insbesondere von etwa 80 bis 500 aufweist.

3. Pharmazeutisches Mittel nach einem der vorhergehenden Ansprüche, **dadurch gekennzeichnet, daß** das Lactalbuminhydrolysat erhältlich ist durch enzymatische Hydrolyse mit Papain, Pan= creatin und wenigstens einer bakteriellen Protease.

4. Pharmazeutisches Mittel nach Anspruch 1, **dadurch gekennzeichnet, daß** die Fraktion des Lactalbuminhydrolysats erhältlich ist durch Extraktion eines Lactalbuminhydrolysats mit Ethanol und Gewinnung des Ethanolextrakts, gewünschtenfalls als Trokkenextrakt.

5. Pharmazeutisches Mittel nach Anspruch 1, **dadurch gekennzeichnet, daß** die Fraktion des Lactalbuminhydrolysats erhältlich ist durch Extraktion eines Lactalbuminhydrolysats mit Ethanol, Einengen des Extrakts zur Trockne, Extraktion des resultierenden Ethanol-Trockenextrakts mit Isopropanol und Gewinnung des Isopropanol-Extrakts, gewünschtenfalls als Trockenextrakt.

6. Pharmazeutisches Mittel nach Anspruch 1, **dadurch gekennzeichnet, daß** die Fraktion des Lactalbuminhydrolysats erhältlich ist durch Extraktion eines Lactalbuminhydrolysats mit Ethanol, Einengen des Extrakts zur Trockne, Extraktion des resultierenden Ethanol-Trockenextrakts mit Isopropanol, Einengen des Extrakts zur Trockne, Aufnahme des resultierenden Isopropanol-Trockenextrakts in Ethanol, Zugabe von Wasser bis auf ein Verhältnis von Ethanol:Wasser von etwa 20:80 bis 60:40, vorzugsweise von etwa 30:70 bis 50:50 und insbesondere von etwa 40:60, Abtrennen des Niederschlags und Gewinnen des wäßrig ethanolische Extrakts, gewünschtenfalls als Trockenextrakt.

7. Pharmazeutisches Mittel nach einem der vorhergehenden Ansprüche, **dadurch gekennzeichnet, daß** der antiphlogistische Wirkstoff ausgewählt ist unter Extrakten und Tinkturen von Kamillenblüten, Bromelaine, Weidenrinde, Arnika, Herba Symphyti, Radix Symphyti, Peregrini recentis und Rosskastaniensamen, Fludrocortison, Dexametason, Hydrocortison, Methylprednisolon, Prednisolon, Prednison, Triamcinolon, Deflazacort, Betamethason, Predmyliden, Budesonid, Chloprednol, Fluorcortolon, Cortison, Rimexolon, Flumetason, Flunisolid, Fluocortinbotyl, Fluocinolon, Fluocortolon, Fluorometholon, Fluticason, Beclomeson, Salicylsäure, Mofebutazon, Kebuzon, Phenazon, Metamizol, Paracetamol, Acemetazin, Ibuprofen, Naproxen, Diclofenac, Indometacin, Lonazolac, Aceclophenac, Fluorbiprofen, Ketoprofen, Naproxen, Mefenaminsäure, Acemetacin, Proglumetazin, Etofenamat, Tiaprofensäure, Dexketoprofen, Azapropazon, Lornoxicam, Meloxicam, Piroxicam, Tenoxicam , Auranophin und Natriumaurothiomalat, Sulfasalazin, Resochin, Chloroquin, Dapson, Methotrexat, Mesalacin, Cinnaricin, Dimethylsulfoxid und Derivaten davon, gegebenenfalls auch in Salzform.

8. Pharmazeutisches Mittel nach Anspruch 7, **dadurch gekennzeichnet, daß** der antiphlogistische Wirkstoff ausgewählt ist unter Prednison, Diclofenac, Ibuprofen, Indometacin und Derivaten davon, gegebenenfalls auch in Salzform.

9. Verwendung wenigstens eines antiphlogistischen Wirkstoffs und eines Lactalbuminhydrolysats oder einer Lactalbuminhydrolysat-Peptide enthaltenden Fraktion davon zur Herstellung eines pharmazeutischen Mittels zur Behandlung von Erkrankungen des rheumatischen Formenkreises.

10. Verwendung nach Anspruch 9, wobei der antiphlogistische Wirkstoff und das Lactalbuminhydrolysat oder die Fraktion davon gleichzeitig oder zeitlich beabstandet, gemeinsam in einer Formulierung oder getrennt in wenigstens zwei verschiedenen Formulierungen, zu verabreichen sind.

## Claims

1. A pharmaceutical composition comprising i) at least one antiinflammatory active ingredient and ii) lactalbumin hydrolysate or a fraction thereof which contains lactalbumin hydrolysate peptides.

2. The pharmaceutical composition as claimed in claim 1, **characterized in that** the lactalbumin hydrolysate or the fraction thereof has a weight average molecular weight of approximately 50 to 1200, preferably of approximately 60 to 800 and in particular of approximately 80 to 500.

3. The pharmaceutical composition as claimed in either of the preceding claims, **characterized in that** the lactalbumin hydrolysate is obtainable by enzymatic hydrolysis with papain, pancreatin and at least one bacterial protease.

4. The pharmaceutical composition as claimed in claim 1, **characterized in that** the lactalbumin hydrolysate fraction is obtainable by extraction of a lactalbumin hydrolysate with ethanol and obtaining the ethanol extract, if desired as dry extract.

5. The pharmaceutical composition as claimed in claim 1, **characterized in that** the lactalbumin hydrolysate fraction is obtainable by extraction of a lactalbumin hydrolysate with ethanol, concentration of the extract to dryness, extraction of the resulting ethanol dry extract with isopropanol and obtaining the isopropanol extract, if desired as dry extract.

6. The pharmaceutical composition as claimed in claim 1, **characterized in that** the lactalbumin hydrolysate fraction is obtainable by extraction of a lactalbumin hydrolysate with ethanol, concentration of the extract to dryness, extraction of the resulting ethanol dry extract with isopropanol, concentration of the extract to dryness, taking up the resulting isopropanol dry extract in ethanol, addition of water until the ethanol:water ratio is approximately 20:80 to 60:40, preferably approximately 30:70 to 50:50 and in particular approximately 40:60, removal of the precipitate and obtaining the hydroethanolic extract, if desired as dry extract.

7. The pharmaceutical composition as claimed in any of the preceding claims, **characterized in that** the antiinflammatory active ingredient is selected from extracts and tinctures of camomile flowers, bromelains, willow bark, arnica, herba symphyti, radix symphyti, Peregrini recentis and horse chestnut seeds, fludrocortisone, dexametasone, hydrocortisone, methylprednisolone, prednisolone, prednisone, triamcinolone, deflazacort, betamethasone, predmylidene, budesonide, chloprednol, fluorocortolone, cortisone, rimexolone, flumetasone, flunisolide, fluocortin botyl, fluocinolone, fluocortolone, fluorometholone, fluticasone, beclomethasone, salicylic acid, mofebutazone, kebuzone, phenazone, metamizole, paracetamol, acemetazin, ibuprofen, naproxen, diclofenac, indometacin, lonazolac, aceclophenac, fluobiprofen, ketoprofen, naproxen, mefenamic acid, acemetacin, proglumetazin, etofenamate, tiaprofenic acid, dexketoprofen, azapropazone, lornoxicam, meloxicam, piroxicam, tenoxicam, auranofin and sodium aurothiomalate, sulfasalazine, resochin, chloroquine, dapsone, methotrexate, mesalazine, cinnarizine, dimethyl sulfoxide and derivatives thereof, optionally also in salt form.

8. The pharmaceutical composition as claimed in claim 7, **characterized in that** the antiinflammatory active ingredient is selected from prednisone, diclofenac, ibuprofen, indometacin and derivatives thereof, optionally also in salt form.

9. The use of at least one antiinflammatory active ingredient and of a lactalbumin hydrolysate or a fraction thereof which contains lactalbumin hydrolysate peptides for preparing a pharmaceutical composition for the treatment of rheumatoid diseases.

10. The use as claimed in claim 9, wherein the antiinflammatory active ingredient and the lactalbumin hydrolysate or the fraction thereof are to be administered simultaneously or with a time lag, together in one formulation or separately in at least two different formulations.

## Revendications

1. Agent pharmaceutique, contenant i) au moins un agent actif anti-inflammatoire et ii) un hydrolysat de lactalbumine ou une de ses fractions contenant des peptides d'hydrolysat de lactalbumine.

2. Agent pharmaceutique selon la revendication 1, **caractérisé en ce que** l'hydrolysat de lactalbumine ou sa fraction présente un poids moléculaire moyen en poids d'environ 50 à 1200, de préférence d'environ 60 à 800 et en particulier d'environ 80 à 500.

3. Agent pharmaceutique selon l'une quelconque des revendications précédentes, **caractérisé en ce que** l'hydrolysat de lactalbumine peut être obtenu par hydrolyse enzymatique avec de la papaïne, de la pancréatine et au moins une protéase bactérienne.

4. Agent pharmaceutique selon la revendication 1, **caractérisé en ce que** la fraction de l'hydrolysat de lactalbumine peut être obtenue par extraction d'un hydrolysat de lactalbumine avec de l'éthanol et obtention de l'extrait éthanolique, si on le souhaite en tant qu'extrait sec.

5. Agent pharmaceutique selon la revendication 1, **caractérisé en ce que** la fraction de l'hydrolysat de lactalbumine peut être obtenue par extraction d'un hydrolysat de lactalbumine avec de l'éthanol, par concentration de l'extrait jusqu'à dessiccation, extraction de l'extrait sec éthanolique qui en résulte avec de l'isopropanol et obtention de l'extrait isopropanolique, si on le souhaite en tant qu'extrait sec.

6. Agent pharmaceutique selon la revendication 1, **caractérisé en ce que** la fraction de l'hydrolysat de lactalbumine peut être obtenue par extraction d'un hydrolysat de lactalbumine avec de l'éthanol, par concentration de l'extrait jusqu'à dessiccation, extraction de l'extrait sec éthanolique qui en résulte avec de l'isopropanol, concentration de l'extrait à dessiccation, recueil de l'extrait sec isopropanolique qui en résulte dans de l'éthanol, addition d'eau jusqu'à un rapport éthanol/eau d'environ 20:80 à 60:40, de préférence d'environ 30:70 à 50:50 et, en particulier, d'environ 40:60, séparation du précipité et obtention de l'extrait éthanolique aqueux, si on le souhaite en tant qu'extrait sec.

7. Agent pharmaceutique selon l'une des revendications précédentes, **caractérisé en ce que** l'agent actif anti-inflammatoire est choisi parmi les extraits de teintures de fleurs de camomille, de bromélaïne, d'écorce de saule, d'arnica, de Symphity herba, de Symphity radix, de Peregrini recentia et de graines de marron, la fludrocortisone, la dexamétasone, l'hydrocortisone, la méthylprednisolone, la prednisolone, la prednisone, la triamcinolone, le déflazacort, la bétaméthasone, la predmylidène, le budesonide, le chloprednol, la fluorcortolone, la cortisone, la rimesolone, le flumetasone, le flunisolide, le fluocortinbotyle, la fluocinolone, le fluocortolone, le fluorométholone, la fluticasone, la béclomésone, l'acide salycilique, la mofébutazone, la kébuzone, la phénazone, la métamizole, le paracétamol, l'acémétazine, l'ibuprofène, le naproxène, le diclofénac, l'indométacine, le lonazolac, l'acéclophénac, le fluorbiprofène, le kétoprofène, le naproxène, le méfénamine acide, l'acémétacine, la proglumétazine, l'étofénamate, le tiaprofène acide, le dexkétoprofène, l'azapropazone, le lornoxicame, le méloxicame, le piroxicame, le ténoxicame, l'auranophine et l'aurothiomalate de sodium, la sulfasalazine, la résochine, le chloroquine, le dapsone, le méthotrexate, la mésalacine, la cinnaricine, le diméthylsulfoxyde et leurs dérivés, le cas échéant éventuellement sous forme de sels.

8. Agent pharmaceutique selon la revendication 7, **caractérisé en ce que** l'agent actif anti-inflammatoire contenu est la prednisone, le diclofénac, l'ibuprofène, l'indométacine et leurs dérivés, le cas échéant éventuellement sous forme de sels.

9. Utilisation d'au moins un agent actif anti-inflammatoire et d'un hydrolysat de lactalbumine ou d'une de ses fractions contenant des peptides d'hydrolysat de lactalbumine pour préparer un agent pharmaceutique pour traiter les maladies de type rhumatoïde.

10. Utilisation selon la revendication 9, l'agent actif anti-inflammatoire et l'hydrolysat de lactalbumine ou bien sa fraction sont à administrer en même temps ou à distance l'un de l'autre, ensemble dans une formulation ou séparément dans au moins deux formulations différentes.
